Europäisches Patentamt

European Patent Office

Office européen des brevets

(19)

(11) Publication number: **0 265 532**
**B1**

(12)

# EUROPEAN PATENT SPECIFICATION

(45) Date of publication of the patent specification:
04.07.90

(51) Int. Cl.⁵: **A61B 5/04**

(21) Application number: **86114678.5**

(22) Date of filing: **22.10.86**

(54) Electrocardiographic electrode pad.

(43) Date of publication of application:
**04.05.88 Bulletin 88/18**

(45) Publication of the grant of the patent:
**04.07.90 Bulletin 90/27**

(84) Designated Contracting States:
**DE FR GB IT**

(56) References cited:
**DE-A- 2 742 058**
**DE-U- 8 502 291**
**NL-A- 6 700 019**
**US-A- 3 788 317**
**US-A- 4 033 334**

(73) Proprietor: **FUKUDA DENSHI CO., LTD., 39-4,**
**Hongo 3-chome Bunkyo-ku, Tokyo 113(JP)**

(72) Inventor: **Inoue, Hiroktsu, 1800-142, Kasoricho,**
**Chiba-shi Chiba-ken(JP)**
Inventor: **Shimizu, Chuji, No. 2-2-3, Narashinodai,**
**Funabashi-shi Chiba-ken(JP)**

(74) Representative: **TER MEER - MÜLLER - STEINMEISTER**
**& PARTNER, Mauerkircherstrasse 45,**
**D-8000 München 80(DE)**

## Description

This invention relates to an electrocardiogram electrode pad for covering an electrocardiogram electrode.

As is well known in the art, electricity is induced in a man with the activity of the heart, brain, muscles, etc. Particularly, electricity induced in the heart can be detected as a weak current induced on the skin surface of the man by an externally installed electrocardiogram to check for any abnormality of the heart. In the use of the electrocardiogram, its input section is electrically coupled to the living body. To this end, its electrode has to be held in close contact with the surface of the skin.

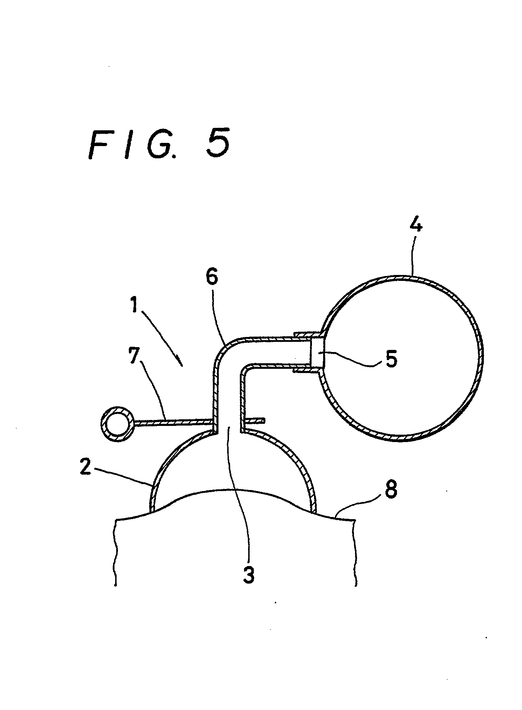

Fig. 5 shows an electrocardiogram electrode held in close contact with the skin of the surface. The electrocardiogram electrode shown in Fig. 5 adopts a suction method for means for obtaining close contact of the electrode with the skin surface of a man. The electrode 1 is made of rubber and has a semispherical cup-like electrode 2 which serves as an electrode section to detect electromotive force generated in a man in contact with the skin surface 8 thereof. The electrode 1 also has a hollow ball-like rubber member 4. The rubber member 4 has an opening 5, which is communicated by a hollow metal tube 6 with a suction port 3 of the cup-like electrode 2. A terminal 7 is connected to the semispherical outer surface of the cup-like electrode 2 and led to the input side of an electrocardiogram (not shown).

To use the prior art rubber suction electrode 1 having the above construction, the rubber member 4 is first compressed with a hand, then the cup-like electrode 2 is held in contact with the skin surface 8 of a man, and then the urging force of the hand is released. As a result, the pressure in the cup-like electrode is reduced by the elastic restoring force of the rubber member 4, whereby the rubber suction electrode 1 is attached by suction to the skin surface.

With the rubber suction electrode 1 held attached by suction to the skin surface, a weak current that is generated in the heart is led to the electrocardiogram to check for any abnormality of the heart. However, this rubber suction electrode 1 provides a considerable suction force. Therefore, after the rubber suction electrode 1 has been removed, its semi-circular trace remains for several days, giving uncomfortability to the patient during this period.

Further, some person may feel ache or inflammation which is sometimes produced on the skin. Further, in case of a person who has much hair on the skin surface, the suction property of the electrode is reduced. Further, where the cup-like electrode 2 is held in contact with the skin surface of a man for the measurement of the weak current, accurate measurement of weak current can not be measured due to a contact resistance generated on the skin of the man. To overcome this drawback, it has been in practice to apply cream or the like to the skin surface so as to reduce the contact resistance, the cup-like electrode 2 being held in contact with the applied cream for the measurement of the weak current. However, it is rather cumbersome and inefficient to apply cream every time the measurement of weak current is done.

The invention has been intended in the light of the above problems in the prior art, and its object is to solve the above problems by fitting an electrode pad as defined in claim 1.

Fig. 1 is a perspective view showing an embodiment of the electrocardiogram electode pad according to the invention;

Fig. 2 is a bottom view showing the embodiment of Fig. 1;

Fig. 3 is a sectional view taken along line III-III in Fig. 1;

Fig. 4 is a view for explaining the method of use of the electrocardiogram electrode pad; and

Fig. 5 is a view for explaining the use of a prior art electrocardiogram electrode pad.

Now, the constitution and functions of the invention will be described with reference to the accompanying drawings.

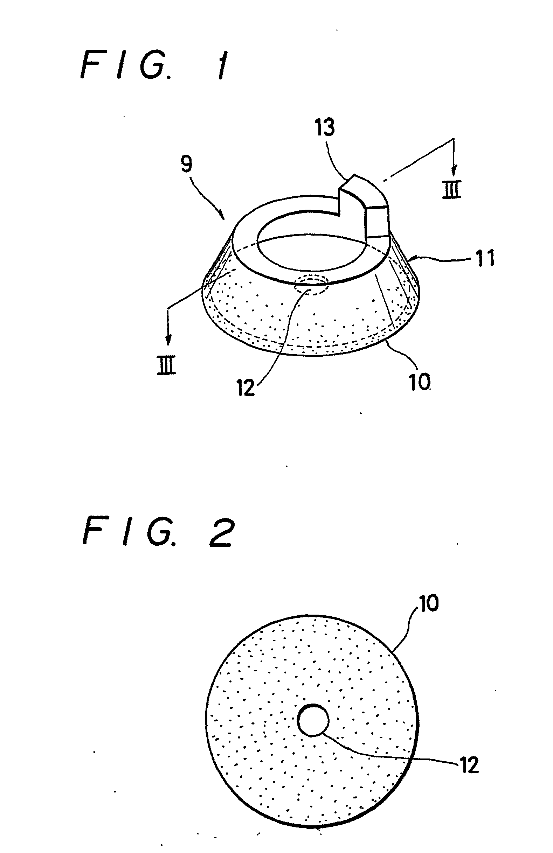

Fig. 1 is a pespective view showing an electrocardiogram electrode pad 9 according to the invention. The electrode pad 9 has a substantially cup-like shape. It is made of water-containing gel of a polyurethane resin. Thus, it has high electric conductivity as well as having flexibility and resiliency. The structure of the electrode pad 9 will now be described in detail. Reference numeral 10 designates a bottom wall of the electrode pad 9. The bottom 10 is substantially circular and is in direct contact with the skin surface of a man.

The electrode pad 9 has a side wall 11 integrally extending from the edge of the bottom 10. The side wall 11 is to be fitted on the electrocardiogram electrode and covers the electrode in co-operation with the bottom wall 10.

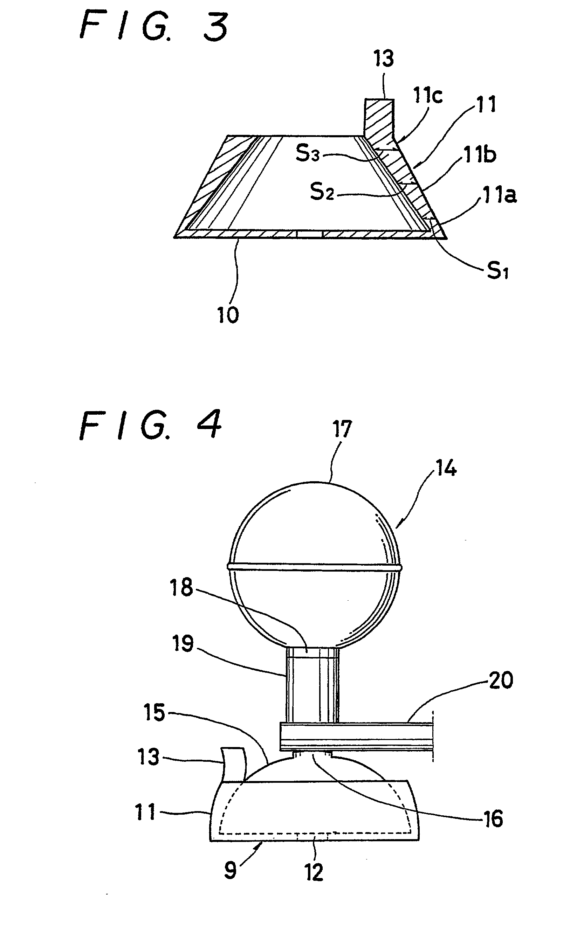

The side wall 11, as shown in Fig. 3, is tapered from its stem portion in the neighborhood of the bottom 10 toward its top 11c. Further, the thickness of the side wall 11 increases as one goes toward the top. That is, the thickness s3 of the top portion 11c is greater than the thicknesses s2 and s1 of inter mediate and stem portions 11b and 11a. Mathematically, $s3 > s2 > s1$

The bottom wall 10 has a substantially central, circular hole 12.

Reference numeral 13 designates a pinch portion 13 projecting from the top of the side wall 11. This pinch portion 13 facilitates the fitting of the electrode pad 9 on the electrocardiogram electrode, as shown in Fig. 4, and removing the former from the latter.

In the illustrated embodiment, the pinch portion 13 is integral with and made of the same material as the electrode pad. However, it is possible to form a pinch portion from a different material from the electrode pad, e.g., a woven sheet, a resin sheet or a rubber sheet. The electrode pad 9 is made of water-containing gel and thus has viscosity. Therefore, where the pinch portion is made of the different material noted above from the electrode pad, the electrode pad can be fitted or removed without possibility of attachment of a viscous matter to fingers and thus in a clean and confortable way by pinching the pinch portion.

Further, the provisions of a pinch portion made from a woven sheet, a resin sheet or a rubber sheet can increase the mechanical strength of the electrode pad 9 which is a soft member. Further, while in this embodiment only a single pinch portion 13 is provided, it is possible to provide two, diametrically opposed pinch portions. Now, the use of the electrode pad 9 will be described. Fig. 4 shows the rubber section electrode 14 with the electrode pad 9 fitted thereon. Referring to the Figure, reference numeral 14 designates the rubber suction electrode. The electrode 14 has a construction, in which a suction port 16 of a semi-spherical electrode section for detecting an electromotive force produced in a man and an opening 18 of a hollow ball-like rubber member 17 are communicated with each other by a hollow metal tube 19, to which a terminal 20 led to the input side of an electrocardiogram (not shown) is connected.

The electrode pad 9 is fitted on the rubber suction electrode 14 having the above construction. The electrode pad 9 can be readily fitted by first fitting the side wall 11 on the side opposite the pinch portion 13 on the end of the electrode section 15 of the rubber suction electrode 14, and then pinching and pulling the pinch roller 13 with the thumb and index finger of the right hand while holding the side wall 11 urged against the electrode section 15 by the thumb and index finger of the right hand. In this way, the electrode pad 9 can be readily fitted on the electrode section 15 because it has flexibility.

The fitted electrode pad 9 will not be occasionally detached from the electrode section 15 for the side wall 11 has a greater thickness s3 for its top portion 11c than the intermediate and stem portions 11b and 11a. A satisfactory fit between the electrode section 15 and electrode pad 9 thus can be obtained. Further, since the thickness s3 is greater than the thicknesses s2 and s1, the mechanical strength of the top portion 11c of the side wall 11 can be improved to facilitate the fitting of the pad 9 on the electrode section 15.

The electrode pad 9 can be readily removed from the electrode section 15 of the rubber section electrode 14 by pinching and pulling the pinch portion 13 to the right with the thumb and index finger of the right hand while holding the rubber member 17 with the thumb and index finger of the left hand.

With the electrode pad 9 thus fitted on the rubber section electrode 14, it is held attached by suction to the skin surface of a man for the measurement of a weak current induced in the man. Since the measurement of weak current is done with the electrode pad 9 interposed between the skin surface and electrode section 15 of the rubber suction electrode 14, no trace of the cup-like electrode section will remain on the skin surface.

As has been described in the foregoing, according to the invention an electrode pad is fitted on the electrode section of an electrocardiogram electrode, so that weak current induced in the man is led to the electrode which is held in close contact with the skin surface of the man through the electrode pad, which has electric conductivity. Thus, the weak current in the man can be accurately detached without need of applying any cream to the skin surface.

Further, since the electrode and skin surface are held in close contact via the electrode pad, no trace of the electrode will remain on the skin surface, or there is no possibility of formation of any inflammation even in case of a person having weak skin.

Further, since the side wall of the pad has a greater thickness for its top portion than the intermediate and stem portions, the fitted part will never be occasionally detaohed from the electrode, and a satisfactory fit between the electrode and pad can be obtained. In consequence, a high quality electrocardiographic waveform can be obtained, and also the reference line of the electrocardiograph can be stabilized to result in less polarization voltage. These properties are desired for the electrocardiographic examinaion.

Further, sinde the mechanical strength of the top portion of the side wall 11 of the pad is improved to facilitate the fitting of the pad on the electrode and thus increase the efficiency of the electrocardiographic examination.

Further, the pinch portion facilitates the operation of fitting the electrode pad on the electrode.

Furthermore, since the pinch portion has both flexibility and elasticity, it permits reliable fitting of the pad on the electrode section.

Moreover, since the electrode pad can be handled by pinching the pinch portion and thus without need of touching the buttom of the pad in contact with the skin surface, which is desired from the standpoint of cleanliness. Further, the presence of the pinch portion increases the mechanical strength of the pad.

## Claims

1. An electric-conductive electrocardiogram electrode pad having elasticity and flexiblity for being fitted on the electrode section of a suction electrocardiogram electrode (2) to be held in close contact with the skin surface of a man to derive a weak current therefrom, said electrocardiographic electrode pad (9) having the shape of a tapered cup and having at its wider end a bottom wall (10) to be held in contact with the skin surface of the man and a side wall (11) extending from the edge of said bottom wall to be fitted on said electrode section of an electrocardiogram section electrode, said bottom wall having a central, circular hole (12), said side wall having an increasing thickness from the edge of the bottom wall towards its free end at the narrower end of the cup shaped pad, said side wall having a pinch portion (13) projecting from the free end thereof to be pinched when fitting or removing said electrocardiogram electrode pad with respect to said electrocardiogram electrode.

2. Electrocardiogram electrode pad as claimed in claim 1, characterized in that said electrode pad (9) and said pinch portion (13) consist of a water-containing gel.

3. The electrocardiogram electrode pad according to claim 1, wherein said pinch portion (13) is made from a woven sheet.

4. The electrocardiogram electrode pad according to claim 1, wherein said pinch portion (13) is made from a resin sheet.

5. The electrocardiogram electrode pad according to claim 1, wherein said pinch portion (13) is made form a rubber sheet.

**Patentansprüche**

1. Elektrisch leitende Elektrokardiogramm-Elektrodenhülle mit Elastizität und Flexibilität zur Anbringung an dem Elektrodenabschnitt einer Elektrokardiogramm-Saugelektrode (2), die in enger Berührung mit der Hautoberfläche eines Menschen zur Ableitung eines schwachen Stroms von dieser zu halten ist, welche elektrokardiografische Elektrodenhülle (9) die Form einer kegelstumpfförmigen Tasse aufweist und eine Bodenwand (10) an irem weiteren Ende besitzt, die in Berührung mit der Hautoberfläche des Menschen zu halten ist, sowie eine Seitenwand (11), die sich vom Rand der Bodenwand erstreckt und auf den Elektrodenabschnitt einer Elektrokardiogramm-Saugelektrode zu bringen ist, welche Bodenwand eine mittlere, kreisförmige Öffnung (12) aufweist, welche Seitenwand eine zunehmende Dicke vom Rand der Bodenwand in Richtung ihres freien Endes am schmaleren Ende der tassenförmigen Hülle besitzt, welche Seitenwand einen Zugbereich (13) aufweist, der vom freien Ende der Seitenwand vorspringt und gezogen wird, wenn die Elektrokardiogramm-Elektrodenhülle auf die Elektrokardiogramm-Elektrode aufgezogen oder von dieser entfernt wird.

2. Elektrokardiogramm-Elektrodenhülle nach Anspruch 1, dadurch gekennzeichnet, daß die Elektrokardiogramm-Elektrodenhülle (9) nd der Zugbereich (13) aus einem Wasser enthaltenden Gel bestehen.

3. Elektrokardiogramm-Elektrodenhülle nach Anspruch 1, bei der der Zugbereich (13) aus einem gewebten Blatt besteht.

4. Elektrokardiogramm-Elektrodenhülle nach Anspruch 1, bei der der Zugbereich (13) aus einem Harz-Blatt besteht.

5. Elektrokardiogramm-Elektrodenhülle nach Anspruch 1, bei der der Zugbereich (13) aus einem Gummi-Blatt besteht.

**Revendications**

1. Coussinet pour électrode électrocardiographique électriquement conductrice possédant une élasticité et une flexibilité pour être adaptée sur la section d'élasticité d'électrode d'une électrode électrocardiographique à aspiration (2) pour être maintenue en contact étroit avec la surface de la peau d'un être humain pour en prélever un courant faible, ledit coussinet d'électrode cardiographique (9) ayant la forme d'une coupe conique et possédant à sont extrémité élargie une paroi inférieure (10) destinée à être maintenue en contact de la surface de la peau d'un être humain et une paroi latérale (11) s'étendant depuis le bord de ladite paroi inférieure destinée à être ajustée sur ladite section d'électrode d'une électrode électrocardiographique à aspiration, ladite paroi de fond possédant un trou circulaire central (12), ladite paroi latérale possédant une épaisseur croissant depuis le bord de la paroi inférieure vers son extrémité libre à l'extrémité étroite du coussinet en forme de coupe, ladite paroi latérale possédant une partie de préhension (13) faisant saillie de son extrémité libre pour être saisie lors de la mise en place ou du retrait dudit coussinet d'électrode électrocardiographique par rapport à ladite électrode électrocardiographique.

2. Coussinet d'électrode électrocardiographique selon la revendication 1, caractérisé en ce que ledit coussinet d'électrode électrocardiographique (9) et ladite partie de préhension (13) consistent en un gel contenant de l'eau.

3. Coussinet d'électrode électrocardiographique selon la revendication 1, dans lequel ladite partie de préhension (13) est réalisée à partir d'une feuille tissée.

4. Coussinet d'électrode électrocardiographique selon la revendication 1, dans lequel ladite partie de préhension (13) est réalisée à partie d'une feuille de résine.

5. Coussinet d'électrode électrocardiographique selon la revendication 1, dans lequel ladite partie de préhension (13) est réalisée à partir d'une feuille de caoutchouc.

EP 0 265 532 B1

# F I G. 1

# F I G. 2

# FIG. 3

# FIG. 4

# F I G. 5